# EUROPEAN PATENT APPLICATION

(11) **EP 1 685 809 A2**
(43) Date of publication of application: **02.08.2006**
(21) Application number: 06002438.7
(22) Date of filing: 07.09.2000
(51) Int. Cl.: A61F 2/32, A61F 2/30

(54) **Combination of material for joint prosthesis**

(30) Priority: 08.09.1999 GB 9921145; 22.06.2000 GB 0015197
(62) Divisional of application: 00958813.8
(71) Applicant: Depuy International Limited, Beeston, Leeds LS11 8DT (GB)
(72) Inventor: Fisher, John, Adel, Leeds LS16 8NY (GB); Farrar, Richard, North Rigton LS17 0DW (GB)
(74) Representative: Belcher, Simon James

(57) **Abstract**

An orthopaedic joint prosthesis comprises first and second articulating components having respective bearing surfaces in contact with one another. The material of the first bearing surface comprises a metallic material and the material of the second bearing surface comprises a ceramic material. The hardness of the metallic material is at least about 2500 MPa, and the hardness of the ceramic material is greater than that of the metallic material by at least about 4000 MPa.

## Description

This invention relates to an orthopaedic joint prosthesis which comprises first and second articulating components having respective bearing surfaces in contact with one another.

Orthopaedic joint prostheses are known for replacement of natural joints such as a hip, knee, ankle and shoulder joint. Joint replacement generally involves removal of tissue from each of the articulating bones of the joint, and implantation of prosthesis components in each of the articulating bones.

It is important that wear of the components of an artificial joint is minimised because wear debris can give rise to adverse physiological reactions. The selection of the materials for the joint components is made in such a way that wear is minimised, and also to ensure that the physiological reaction to such wear debris as is created is minimised.

It is common for a joint prosthesis to comprise components whose mating articulating surfaces comprise a metal and a polymer respectively (a "metal-on-polymer" joint). For example, the bearing surface on one of the components might comprise a cobalt-chrome or steel-based alloy and the corresponding bearing surface on the other component might comprise a high molecular weight polyethylene. The surfaces of both components are finished so that they are smooth. During articulation of the joint, the polymeric component is subject to gradual wear, forming fine particles of the polymer.

Attempts have been made to form joint prostheses in which both of the articulating surfaces are manufactured from metals (a "metal-on-metal" joint). Even with very fine finishing to produce smooth bearing surfaces, wear in such prostheses cannot be avoided. The wear inevitably gives rise to the formation of metal particles. The metal particles resulting from the wear tend themselves to have abrasive properties so that, when present between the articulating surfaces, they can give rise to further wear. Furthermore, because of the similar hardnesses of the two articulating surfaces (because both are formed from metals), the wear tends to take place on both surfaces. Although the wear volumes in a metal-on-metal prosthesis are less than in a metal-on-polymer prosthesis, the metallic wear particles are very small (for example about 100 nm) and consequently high in number. The physiological effects of metal wear particles are not fully understood. However, there clearly remains a desire to reduce the volume and number of wear particles further compared with some existing systems.

The present invention provides an orthopaedic joint prosthesis in which the material of one of the bearing surfaces comprises a metallic material and the material of the other bearing surface comprises a ceramic material.

Accordingly, in one aspect, the invention provides an orthopaedic joint prosthesis which comprises first and second articulating components having respective bearing surfaces in contact with one another, the material of the first bearing surface comprising a metallic material and the material of the second bearing surface comprising a ceramic material, the hardness of the metallic material being at least about 2500 MPa, and the hardness of the ceramic material being greater than that of the metallic material by at least about 4000 MPa.

The use of bearing surfaces formed from metallic and ceramic materials gives rise to significant advantages. The materials allow the formation of very smooth bearing surfaces with very accurately controlled geometry so that the surfaces can be accurately matched. Materials of the two surfaces can be selected with hardnesses that are greater than those in other joint systems so that the tendency for them to wear during articulation is reduced, and with a differential hardness which can ensure that one of the surfaces is generally able to remain smooth during articulation. This in turn can result in low wear of the opposite surface.

The ceramic material of the second bearing surface is harder than the metallic material of the first bearing surface. For example, it can be preferred for the ratio of the hardness of the ceramic material of the second bearing surface to that of the metallic material of the first bearing surface to be at least about 2, more preferably at least about 3. It can be preferred for the difference between the hardness of the ceramic material of the second bearing surface and that of the metallic material of the first bearing surface to be at least about 8000 MPa, more preferably at least about 10000 MPa. The difference will often be not more than about 30000 MPa, for example not more than about 20000 MPa or 15000 MPa. Hardnesses are measured according to DIN50359.

Use of a ceramic material that is significantly harder than the metallic material has the advantage that the tendency of the ceramic material to wear during articulation is minimised. Furthermore, the action of the ceramic surface against the metallic surface can polish the metallic surface, so that further wear of the articulating surfaces is kept to a minimum.

Preferably, the hardness of the ceramic material of the second bearing surface is at least about 10000 MPa, more preferably at least about 14000 MPa, for example at least about 15000 MPa, or at least about 20000 MPa or higher.

Preferably, the hardness of the metallic material of the first bearing surface is at least about 3500 MPa. The hardness of the metallic material will often be less than about 8000 MPa, especially less than about 7000 MPa, for example in the range 4000 to 5000 MPa.

The ceramic material will generally comprise a dense, hard, crystalline, non-metallic material, especially an inorganic material. A bearing material might be formed from such a material by a process which includes, for example, exposure to high temperature and pressure. Examples of ceramic materials include hard oxides. Examples of suitable hard oxide materials include aluminium oxide (aluminia) and zirconium oxide (zirconia). Zirconium oxide will preferably be used in mixtures with other materials such as one or more of aluminium oxide and yttrium oxide. Other ceramic materials include certain carbides and nitrides, such as carbides and nitrides of titanium, chromium, silicon, aluminium and zirconium.

Metals which can be used to form orthopaedic joint prostheses are known and can be used in the joint prosthesis of the present invention. Examples of such metals include cobalt chrome alloys, titanium alloys, and certain stainless steels.

Preferably, the surface roughness of the ceramic bearing surface is not more than about 0.015 µm Rₐ, more preferably not more than about 0.01 µm Rₐ, especially not more than about 0.008µm Rₐ, for example not more than about 0.005 µm Rₐ, as measured using conventional surface profilometer apparatus. It is an advantage of the use of ceramic materials in the joint prosthesis of the present invention that they can conveniently be finished with such smooth surface properties by readily accessible surface finishing techniques using commercially available equipment.

Preferably, the surface roughness of each of the bearing surfaces is not more than about 0.05 µm Rₐ, more preferably not more than about 0.03 µm Rₐ, for example not more than about 0.01 µm Rₐ.

The joint prosthesis of the present invention can comprise first and second components of which one has a generally rounded convex bearing surface, and the other is concave and can receive the component with the convex bearing surface within it. Some such concave components are sometimes referred to as "cup components". Examples of joint prostheses with these features include hip and shoulder joint prostheses. In many products, the rounded bearing surfaces will be substantially spherical, although it will be understood that the surface will generally have the configuration of only a part of a sphere. Preferably, the component with the convex bearing surface is the second component and has a ceramic bearing surface, the concave component having a metallic bearing surface, although alternative constructions can be used in which the component with the convex bearing surface is the first component with a metallic bearing surface.

When one of the components has a rounded (especially spherical) bearing surface, its diameter can be up to 60 mm or more. However, it can be preferred for the diameter to be not more than about 40 mm, more preferably not more than about 35 mm (for example about 28 mm), and can be not more than about 20 mm, for example not more than about 15 mm, and even as low as about 10 mm. It is an advantage of the use of the combination of hard materials used in the prosthesis of the present invention, and of their combined resistance to wear, that they allow the bearing surfaces to be created with smaller transverse dimensions (diameter in the case of a spherical bearing surface) than has been found to be necessary when other bearing surface materials are used.

When the bearing surfaces are spherical, it is preferred that their diameters are controlled so that variations do not exceed about 0.02 mm, more preferably about 0.015 mm, especially about 0.005 mm. In the case of the ceramic component at least, it is possible for variations in the diameter to be kept below about 0.002 mm, for example to about 0.001 mm.

An example of a technique which can be used to provide a component with a bearing surface which is spherical and polished involves spinning the component about its axis against a cylindrical abrading stone, itself spinning around its axis. The component and the abrading stone are arranged with the angle between their axes about 45°, and so that their axes intersect at the centre of the sphere on the component. The combined rotation of the component and the abrading stone creates a spherical surface provided that the circle of contact covers both the pole and the equator of the sphere. A final polishing step can be included to optimise the surface finish.

The cup component can have a concave internal bearing surface with generally spherical configuration. It is known for such bearing surfaces to have configurations which deviate from accurate sphericity, and such deviations might be incorporated into the joint prosthesis of the present invention.

The first and second components of the prosthesis of the invention can be made entirely of the metallic and ceramic materials which provide the bearing surfaces. Frequently however it will be preferred for the bearing surface materials to provide just a part of the components. For example, in the case of the femoral component of a hip joint prosthesis. the spherical head (which is received in the acetabular cup component) might be formed as a solid block of ceramic material, with a tapered hollow formed in it which can receive a tapered pin on the main body part of the femoral component. The body part, with the stem which extends into the medullary cavity of the femur, can be formed from a metallic material as is known.

At least one of the components of the prosthesis might be provided with the bearing material provided as a surface layer. For example, the second component might be formed generally from one or more metallic materials, with a surface layer of a ceramic material which is to provide the bearing surface. The surface layer will preferably be at least about 0.005 mm, more preferably at least about 0.01 mm, especially at least about 0.05 mm, for example at least about 0.1 mm, 0.5 mm, 1.0 mm, 1.5 mm or 2.0 mm or more. It will be understood that the surface coating of the ceramic material is such that it has to be specifically created on the component, in contrast to surface coatings which are formed by processes such as oxidation due to exposure to environmental oxidising agents. The hardness of a surface layer is measured according to DIN50359.

One or both of the articulating components can be manufactured as separate parts from which the components can then be assembled. For example, when the prosthesis is for use in a patient's hip, the femoral component can be manufactured as separate stem and head parts, with the head providing the bearing surface. Similarly, the acetabular component can be manufactured as separate shell and insert (or liner) parts with the insert providing the corresponding bearing surface. Separate parts of the components can be fixed together using known techniques, including mechanical fixation techniques (especially relying on frictional forces between corresponding tapered surfaces).

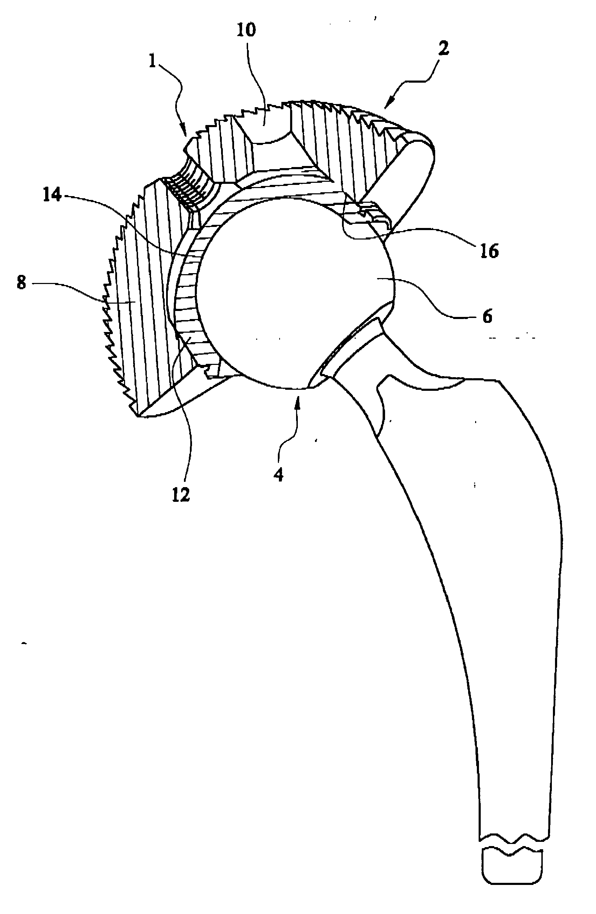

An embodiment of a joint prosthesis according to the present invention will now be described by way of example with reference to the accompanying drawing, which is a side view, partially in section, through the prosthesis.

Referring to the drawing, a hip joint prosthesis 1 comprises an acetabular component 2 and a femoral component 4. The femoral component includes a head 6 which has a spherical convex bearing surface. The acetabular component comprises a metal shell 8 with holes 10 extending through it to accommodate screws by which the shell can be fastened into a patient's pelvis.

The acetabular component contains an insert 12 which has a spherical concave bearing surface 14. The insert and shell have corresponding tapered surfaces 16 which mate together when the insert is inserted into the shell. The insert is held in place within the shell by frictional forces between the tapered surfaces.

The head 6 of the femoral component is formed from medical grade alumina with a hardness of about 16000 MPa. The surface roughness (Rₐ) of the head is less than about 0.01 µm. The insert is formed from medical grade high carbon cobalt-chrome alloy, with a similar surface roughness and a hardness of about 5000 MPa. The diameters of the head of the femoral component and the insert are about 28 mm, with a diametral clearance of 60 µm. The hardnesses of the materials was measured according to DIN50359 using a Fisher 4100 system.

A joint prosthesis as described above was compared with a prosthesis in which the head of the femoral component was made from a low carbon cobalt-chrome alloy instead of alumina to enable comparisons to be made of relative wear rates. The prostheses were tested in an appropriate anatomical position in a hip simulator with two axes of motion and one axis of loading, as described by Barbour *et al* in Proc Instn Mech Engnrs, Part H, Vol 213 (1999). The articulation and loading patterns were in line with what is described by Paul in Proc Instn Mech Engnrs, Vol 181, Part 3J, pp 8-15 (1967). The prostheses were lubricated using bovine serum (25%). The prostheses were tested for 5 million cycles. The articulation was interrupted from time to time to collect the lubricant and any wear debris for analysis.

It was found that the wear debris from the ceramic-on-metal prosthesis according to the present invention was found to be significantly less than from the prosthesis with metal-on-metal bearing surfaces. The metal-on-metal prosthesis showed a high "bedding-in" wear rate (3.12 ± 0.45 mm³/10⁶ cycles) for about the first million cycles, which then settled down to a lower steady state wear rate (1.56 ± 0.78 mm³/10⁶ cycles). Very low wear was detected on the ceramic-on-metal components, amounting to about 0.01 mm³/10⁶ cycles over the course of the entire 3 million cycle test, although the precise amount of wear could not be measured accurately using available weighing apparatus. Furthermore, substantially all of the wear debris from the ceramic-on-metal components was metallic.

The mean sizes of the particles of wear debris were 18 ± 1.37 nm for the ceramic-on-metal components, and 30 ± 2.25 nm for the metal-on-metal components.

## Claims

1. An orthopaedic joint prosthesis which comprises first and second articulating components having respective bearing surfaces in contact with one another, the material of the first bearing surface comprising a metallic material and the material of the second bearing surface comprising a ceramic material, the hardness of the metallic material being at least about 2500 MPa, and the hardness of the ceramic material being greater than that of the metallic material by at least about 4000 MPa.

2. A prosthesis as claimed in claim 1, in which the ratio of the hardness of the ceramic material of the second bearing surface to that of the metal of the first bearing surface is at least about 2.

3. A prosthesis as claimed in claim 2, in which the said hardness ratio is at least about 3.

4. A prosthesis as claimed in claim 1, in which the difference between the hardness of the ceramic material of the second bearing surface and that of the metal of the first bearing surface is not more than about 30000 MPa.

5. A prosthesis as claimed in claim 1, in which the hardness of the ceramic material of the second bearing surface is at least about 10000 MPa.

6. A prosthesis as claimed in claim 1, in which the ceramic material comprises a hard oxide.

7. A prosthesis as claimed in claim 1, in which one of the components has a substantially convex bearing surface, and the other of the components is concave and can receive the component with the convex bearing surface within it.

8. A prosthesis as claimed in claim 7, in which the second component has the convex bearing surface and the first component is concave.

9. A prosthesis as claimed in claim 1, in which the material of the bearing surface of at least one of the components is provided by a surface layer,

10. A prosthesis as claimed in claim 9, in which the thickness of the surface layer is at least about 50 µm.

11. A prosthesis as claimed in claim 1, in which the surface roughness of each of the bearing surfaces is not more than about 0.05 µm.
